# EUROPEAN PATENT APPLICATION

(11) **EP 2 833 312 A1**
(43) Date of publication of application: **04.02.2015**
(21) Application number: 12872645.2
(22) Date of filing: 30.03.2012
(51) Int. Cl.: G06Q 50/02

(54) **ESTRUS NOTIFICATION METHOD, ESTRUS NOTIFICATION DEVICE AND ESTRUS NOTIFICATION PROGRAM**

(71) Applicant: FUJITSU LIMITED, Kawasaki-shi Kanagawa 211-8588 (JP)
(72) Inventor: UCHINO, Tetsuya, Fukuoka-shi Fukuoka 814-8589 (JP); MOTOSHIMA, Toshimi, Fukuoka-shi Fukuoka 814-8589 (JP); KANAMORI, Akihito, Fukuoka-shi Fukuoka 814-8589 (JP); INENAGA, Mitsuhisa, Fukuoka-shi Fukuoka 814-8589 (JP); WATANABE, Katsuyoshi, Fukuoka-shi Fukuoka 814-8589 (JP); YAMANO, Daiji, Kawasaki-shi Kanagawa 211-8588 (JP)
(74) Representative: Fenlon, Christine Lesley
(86) International application number: PCT/JP2012/058750
(87) International publication number: WO 2013/145321

(57) **Abstract**

An estrus notifying apparatus (104) receives from a relay device (102), measurement result information of a step count transmitted by a communications device (101) attached to a livestock animal (A). The estrus notifying apparatus (104) determines based on the measurement result information, whether the livestock animal (A) is in an abnormal state. The estrus notifying apparatus (104), upon determining an abnormal state and receiving from a worker (W), notification that the livestock animal (A) will be attended to, sets the communications device (101) of the livestock animal (A) to be a tracking target. The estrus notifying apparatus (104), via a relay device (102), transmits to the communications device (101), a change instruction to set the transmission interval for the measurement result information to be short. The communications device (101) shortens the transmission interval based on the received change instruction. Thereafter, the estrus notifying apparatus (104) notifies a worker (W) of position information correlated with the new relay device (102), each time the relay device (102) used to report measurement result information by the communications device (101) set to be a tracking target changes.

## Description

### TECHNICAL FIELD

The present invention related to an estrus notifying method, an estrus notifying apparatus, and an estrus notifying program.

### BACKGROUND ART

A technique of attaching a pedometer to livestock such as grazing cattle and measuring the number of steps walked to detect changes in the state of the animal, such as the onset of estrus or illness is known. Based on the detection of a change in the state of the animal via the pedometer, a worker makes a determination such as whether to mate the animal, administer medical treatment, etc.

Patent Document 1: Japanese Laid-Open Patent Publication No. 2005-32208
Patent Document 2: Japanese Laid-Open Patent Publication No. H8-275243
Patent Document 3: Japanese Laid-Open Patent Publication No. 2007-257481

### DISCLOSURE OF INVENTION

### PROBLEM TO BE SOLVED BY THE INVENTION

Nonetheless, with the conventional techniques, even if state changes are detected from the step count of the animal measured by the communications device attached to the animal, it may take some time before the worker finds the animal, whose state has changed. Therefore, a problem arises in that when the worker goes to the animal, the animal may have moved and thus, cannot be found at the location first notified. In such a case, the worker has to search the pasture area until the animal whose state has changed is found. Consequently, although shortening the interval of the position report of the communications device attached to the animals may be considered, a problem arises in that the battery is consumed.

To solve the problems of the conventional techniques above, one object of the present invention is to provide an estrus notifying method, an estrus notifying apparatus, and an estrus notifying program that can support the tracking of a specific livestock animal.

### MEANS FOR SOLVING PROBLEM

To solve the problems above and achieve an object, according to one aspect of the present embodiment, an estrus notifying method, an estrus notifying apparatus, and an estrus notifying program are proposed that give notification of estrus, based on a step count measurement result that is reported through a relay device, at a first transmission interval, and by a step counting device attached to each livestock animal among plural livestock animals. The estrus notifying method, the estrus notifying apparatus, and the estrus notifying program receive the step count measurement result of the livestock animal and information of the relay device used to report the step count measurement result; determine with respect to each livestock animal and based on the step count measurement result, whether the livestock animal is in estrus; notify a worker of identification information of a specific livestock animal determined to be in estrus and position information correlated with a specific relay device used to report the step count measurement result of the specific livestock animal; set a specific step counting device that is attached to the specific livestock animal, to be a tracking target, when notification of attending to the specific livestock animal is received from the worker; transmit to the specific step counting device, a change instruction to change a reporting interval for the step count measurement result, to a second transmission interval that is shorter than the first transmission interval; and notify the worker of position information correlated with a new relay device, each time the specific relay device used by the specific step counting device to report the step count measurement result changes.

### EFFECT OF THE INVENTION

According to one aspect of the present invention, the tracking of a specific livestock can be supported.

### BRIEF DESCRIPTION OF DRAWINGS

FIG. 1 is a diagram depicting one example of an estrus notifying method according to an embodiment;
FIG. 2 is a diagram depicting an example of system configuration of an estrus notifying system 200;
FIG. 3 is a block diagram depicting a hardware configuration example of a communications device of the present embodiment;
FIG. 4 is a block diagram depicting a hardware configuration example of a relay device 102;
FIG. 5 is a block diagram depicting a hardware configuration example of an estrus notifying apparatus 104, etc.;
FIG. 6 is a diagram depicting an example of the contents of a measurement result information table 201;
FIG. 7 is a diagram depicting an example of the contents of a step count DB 202;
FIG. 8 is a diagram depicting an example of the contents of a transmission source DB 203;
FIG. 9 is a diagram depicting an example of the contents of a relay device DB 204;
FIG. 10 is a diagram depicting an example of the contents of a threshold DB 205;
FIG. 11 is a diagram depicting an example of the contents of a step count cumulation table 1100;
FIG. 12 is a block diagram depicting a functional configuration example of the estrus notifying apparatus 104;
FIG. 13 is a diagram depicting an example of display by a client apparatus 105;
FIG. 14 is a flowchart depicting an example of a process procedure of a communications device 101;
FIG. 15 is a flowchart (part 1) depicting an example of a process procedure of the estrus notifying apparatus 104;
FIG. 16 is a flowchart (part 2) depicting an example of the process procedure of the estrus notifying apparatus 104; and
FIG. 17 is a flowchart (part 3) depicting an example of the process procedure of the estrus notifying apparatus 104.

### BEST MODE(S) FOR CARRYING OUT THE INVENTION

Embodiments of an estrus notifying method, an estrus notifying apparatus, and an estrus notifying program will be described in detail with reference to the accompanying drawings.

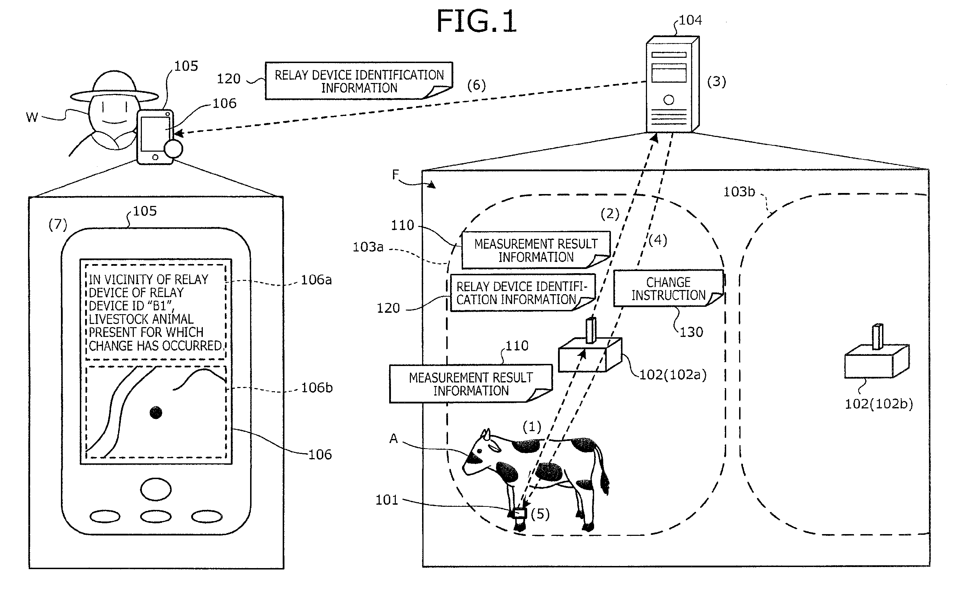

FIG. 1 is a diagram depicting one example of the estrus notifying method according to the present embodiment. In FIG. 1, livestock animals A are raised within the premises of a farm F managed by a worker W. Here, the worker W is one who is engaged in the livestock industry. The farm F is a facility that has a pasture area for pasturing the livestock animals A. A livestock animal A is an animal that can move within the premises of the farm F, for example, within the pasture area of the farm F. For example, a livestock animal A is an animal that moves by ambulation, such as a cow, pig, horse, etc.

A communications device 101 is attached to each livestock animal A. Here, the communications device 101 is a portable computer that measures a step count of the livestock animal A. For example, when the livestock animal A walks, the livestock animal A steps with the right-front leg, sending the leg out toward the ground in the direction of travel. Upon planting the right-front leg on the ground, next, the livestock animal A steps with the left-front leg, sending the leg out toward the ground in the direction of travel. Upon planting the left-front leg on the ground, again, the livestock animal A steps with the right-front leg, and repeats this series of actions. In other words, the step count of the livestock animal A can be the number of times that the livestock animal A steps with the right-front leg or the left-front leg, sending the leg out toward the ground in the direction of travel.

For example, when the state of the livestock animal becomes an abnormal state, which differs from a normal state, consequent to a state change of the livestock animal A, such as the onset of estrus or illness, giving birth, the appearance of a natural predator, etc., the step count per unit time of the livestock animal A increases or decreases compared to that for the normal state. Here, estrus is an excited state accompanying reproductive activity of the livestock animal A. Illness is a state in which the physical or emotional state of the livestock animal A is poor or unfavorable.

When an abnormal state of the livestock animal A arises, the change in the step count of the livestock animal A is used to measure the step count of the livestock animal A, whereby the worker W can know that a change has occurred with the livestock animal A from the step count measurement result.

The communications device 101 is capable of communicating with multiple relay devices 102 installed within the premises of the farm F. Here, a relay device 102 is a computer that is capable of communicating with the communications device 101 and an estrus notifying apparatus 104 described hereinafter. Each of the relay devices 102 is installed at a different position in the farm F. The communications device 101, via a communicable relay device 102, transmits measurement result information that indicates measurement results to the estrus notifying apparatus 104.

For example, if the communications device 101 is located in a communications area 103a of a relay device 102a indicated as an example in FIG. 1 by reference numeral 102a, the communications device 101 can communicate with the relay device 102a. Further, if the communications device 101 is located in a communications area 103b of a relay device 102b indicated as an example in FIG. 1 by reference numeral 102b, the communications device 101 can communicate with the relay device 102b. A communications area, for example, is a 150-meter radius around the relay device 102. The relay device 102, upon receiving measurement result information 110 from the communications device 101, transmits the received measurement result information 110 to the estrus notifying apparatus 104.

The estrus notifying apparatus 104 is a computer that is connected to the relay devices 102, and via the relay devices 102, is able to receive measurement result information 110 of the communications devices 101. Further, for example, the estrus notifying apparatus 104 can be communicably connected to the client apparatus 105, via a given line.

The client apparatus 105 is a portable computer having a display 106 and capable of communicating with the estrus notifying apparatus 104. The client apparatus 105, upon receiving information from the estrus notifying apparatus 104, displays images based on the received information, on the display 106.

Here, as a technique of finding an abnormal state of a pastured livestock animal A, for example, an example will be given describing a technique of finding an estrus state of a female cow. For example, the worker W periodically goes out to the pasture area, and actually visually confirms whether a female cow in estrus is present. Since the worker W has to make search rounds in the farm F, which is extremely large covering several 100 meters to several kilometers on a side, this technique, for example, not only takes time, but also is a burden to the worker W and ultimately, has a high personnel cost. Therefore, although mating implemented by the worker W during pasturing may be foregone, no mating of a female cow in estrus, i.e., not implementing mating when the potential of fertilization is high is a financial loss.

Further, when pasturing female cows, the worker W may also pasture stud bulls for natural mating to occur. However, natural mating by a stud bull is not desirable from the viewpoint of current livestock management ideals of producing better offspring. Furthermore, since bulls are aggressive, only an experienced worker W can handle the bulls and thus, is not an effective technique.

Further, a system is present that uses the property that the step count increases for a female cow in estrus, and attaches to a female cow, a pedometer that measures step count. In this system, the pedometer attached to the female cow transmits measurement results to a server, at given transmission intervals, via a relay device installed in the pasture area. Thus, by notifying the worker W of the relay device that relayed the measurement results, the worker W can surmise the position of the female cow in estrus.

Nonetheless, with the above system, the object of the system itself is to detect female cow estrus. Therefore, the pedometer, for example, is set to transmit measurement results at one-hour intervals. On the other hand, the worker W may be involved with other work such as cleaning the cattle shed and thus, cannot immediately go to the vicinity of the relay device (i.e., cow in estrus). In such a case, when the worker W goes to the vicinity of the relay device, the cow in estrus, which moves moment to moment, may have moved from the vicinity of the relay device and in the end, the worker W has to search the pasture area, which a burden on the worker W.

Further, to know the position of a female cow in estrus, although a technique of attaching a communications apparatus having a GPS function to each female cow may be considered, to attach a communications apparatus having a GPS function to each female cow requires a large cost. Further, the GPS function continuously emits an electronic signal and therefore, the consumption of the battery, which is the driving source, is fast, requiring frequent replacement of the battery or replacement of the communications apparatus itself. Consequently, this technique not only has a high initial cost but also places a burden on the worker W in terms of battery or apparatus replacement and therefore, is not suitable for actual implementation.

Thus, in the estrus notifying method of the present embodiment, the estrus notifying apparatus 104 shortens the transmission interval of the communications device 101, at which measurement results of step counts of the livestock animals are transmitted, if a livestock animal A indicates estrus and the worker goes to track the livestock animal A. Each time the communications device 101 performs communication at the shortened transmission interval, the estrus notifying apparatus 104 determines whether a change has occurred with the identification information (e.g., relay device ID) of the relay device that relayed the communication. When a change has occurred with the identification information (e.g., relay device ID) of the relay device, the estrus notifying apparatus 104 notifies the worker W of the communications area that corresponds to the relay device identification information after the change. Thus, the estrus notifying method of the present embodiment can effectively suppress battery consumption of the communications device 101 and grasp the position of a livestock animal A indicating estrus.

Hereinafter, one example of the estrus notifying method of the present embodiment will be described. In the embodiment, estrus will be taken as one example of an abnormal state of the livestock animal A. Further, in the present embodiment, the livestock animal A will be described as a female cow. Female cows have a characteristic in that the when in estrus, the female cow has a step count that increases per unit time compared to periods when not in estrus and this characteristic of female cows is used.
(1) The communications device 101 measures the step count of the livestock animal A and at a first transmission interval, transmits the measurement result information 110 to a relay device 102. For example, here, the first transmission interval is a one-hour interval. In other words, the first transmission interval is an interval for reporting measurement result information of a livestock animal A.
   Thus, during a period set such that measurement result information is transmitted at the first transmission interval, the communications device 101 sets the transmission frequency of the measurement result information to be lower compared to a period set such that measurement result information is transmitted at a second transmission interval described hereinafter.
(2) The relay device 102, upon receiving the measurement result information 110 from the communications device 101, transmits the received the measurement result information 110 and relay device identification information 120 thereof to the estrus notifying apparatus 104. Here, relay device identification information is information that can identify the relay device 102. For example, relay device identification information is information indicating the relay device ID specific to each relay device 102.
(3) The estrus notifying apparatus 104, upon receiving the measurement result information 110 from the relay device 102, determines based on the received measurement result information 110, whether the step count of the livestock animal A satisfies a given condition. More specifically, for example, in a case where a one-hour step count of the livestock animal A is greater than or equal to a threshold, the estrus notifying apparatus 104 determines that the step count of the livestock animal A satisfies the given condition. The threshold, for example, is set to a value that enables determination that a livestock animal A indicates estrus when the one-hour step count of the livestock animal A is greater than or equal to the threshold. In other words, the estrus notifying apparatus 104 can determine the presence/absence of estrus of a livestock animal A by determining whether the step count of the livestock animal A satisfies a given condition.
   If the step count of the livestock animal A is determined to satisfy the given condition, the estrus notifying apparatus 104 notifies the worker W of the identification information of the livestock animal A indicating estrus and position information correlated with the relay device 102 used to report the measurement result information of the livestock animal A. The identification information of the livestock animal A, for example, is the name of the livestock animal A, identification information of the communications device 101 attached to the livestock animal A, etc. Further, the position information correlated with the relay device 102, for example, is installation position information indicating the installation position of the relay device 102. More specifically, for example, the estrus notifying apparatus 104 gives notification to the worker W by transmitting to the client apparatus 105 possessed by the worker W, the identification information of the livestock animal A indicating estrus and the position information correlated with the relay device 102 used to report the measurement information of the livestock animal A.
   Thereafter, upon receiving from the worker W, notification of attending to the livestock animal A, the estrus notifying apparatus 104 sets the communications device 101 attached to the livestock animal A to a tracking target. More specifically, for example, upon receiving from the client apparatus 105, notification indicating the start of tracking of the livestock animal, the estrus notifying apparatus 104 sets the communications device 101 attached to the livestock animal A to be a tracking target.
(4) When setting the communications device 101 to be a tracking target, the estrus notifying apparatus 104 transmits to the communications device 101, via a relay device 102, a change instruction 130 instructing that the interval for reporting the measurement result information of the livestock animal A be changed to the second transmission interval, which is shorter than the first transmission interval. Here, for example, the second transmission interval is a 10-minute interval.
(5) Upon receiving the change instruction 130 from the estrus notifying apparatus 104, via the relay device 102, the communications device 101 changes the transmission interval for the measurement result information 110 according to the received the change instruction 130. For example, as described above, if the change instruction instructs that the transmission interval be set to a 10-minute interval, the communications device 101 changes the transmission interval for the measurement result information 110 to a 10-minute interval. Thereafter, the communications device 101 transmits the measurement result information 110 at 10-minute intervals.
   Thus, when no estrus of a livestock animal A is detected, the estrus notifying apparatus 104 transmits measurement result information to the communications device 101, at one-hour intervals. When estrus of a livestock animal A is detected and notification indicating that livestock animal A will be attended to is received from the worker W, the estrus notifying apparatus 104 transmits measurement result information to the communications device 101, at 10-minute intervals. Thus, when no estrus of a livestock animal A is detected, the estrus notifying apparatus 104 can suppress the battery consumption of the communications device 101 compared to a case where estrus of a livestock animal A is detected. Further, even if estrus of a livestock animal A is detected, the battery consumption in a case where the worker W cannot track the livestock animal A can be suppressed.
(6) The estrus notifying apparatus 104 notifies the worker W of the position information correlated with the new relay device 102, each time the relay device 102 used to report the measurement result information of the communications device 101 set to be a tracking target changes.
   More specifically, for example, the estrus notifying apparatus 104 receives, via the relay device 102, the measurement result information 110 from the communications device 101 set to be a tracking target. The estrus notifying apparatus 104 compares the relay device identification information 120 of the relay device 102 that is the transmission source of the received measurement result information 110 and the relay device identification information 120 of the relay device 102 from which the previous measurement result information 110 was transmitted and received. If the relay device identification information 120 differs, the estrus notifying apparatus 104 transmits the current relay device identification information 120 to the client apparatus 105. The relay device identification information 120 includes, for example, the relay device ID of the relay device 102, installation position information, communications area information, etc. Thus, the estrus notifying apparatus 104 can efficiently notify the worker W near which relay device the livestock animal A in estrus is currently located.
(7) The client apparatus 105 displays on the display 106, an image based on the relay device identification information 120, each time the relay device identification information 120 is received from the estrus notifying apparatus 104. For example, as indicated by reference numeral 106a in FIG. 1, the client apparatus 105 displays the relay device ID and installation position information (e.g., reference numeral 106b in FIG. 1) of the relay device indicated by the relay device identification information 120.

Thus, the worker W, knowing where the relay device 10 of the relay device ID displayed on the display 106 is, can surmise where the livestock animal A is that has the communications device 101 being tracked. Therefore, when looking for the livestock animal A having the communications device 101 being tracked, the worker W can narrow down the range to be searched, enabling the work load and the time consumed for looking for the livestock animal A to be reduced.

Further, by an image based on installation position information such as that indicated by reference numeral 106b, even an inexperienced worker W who does not know the position of the relay device 102 from the relay device ID can surmise where the livestock animal A having the communications device 101 being tracked is. Therefore, when looking for the livestock animal A having the communications device 101 being tracked, the worker W can narrow down the range to be searched without the range being affected by movement of the livestock animal A, enabling the work load and time consumed in searching for the livestock animal A to be reduced.

As described, in the estrus notifying method of the present embodiment, the estrus notifying apparatus 104 shortens the communications device 101 transmission interval at which the measurement result for the step count of the livestock animal A is transmitted, when the livestock animal A indicates estrus and the worker W has started tracking. Each time the communications device 101 performs communication at the shortened transmission interval, the estrus notifying apparatus 104 compares the identification information (e.g., relay device ID) of the relay device that relayed the current communication and the identification information (e.g., relay device ID) of the relay device that relayed the previous communication and if the identification information differs, that is, when the livestock animal A has moved, the estrus notifying apparatus 104 can notify the worker W of the change in position.

Thus, the estrus notifying method of the present embodiment can suppress battery consumption of the communications device 101 and facilitate knowing the current position of a livestock animal A in estrus. Further, the estrus notifying method of the present embodiment, for example, compared to a case where each female cow has a communications apparatus equipped with a GPS function, the initial cost can be suppressed and since power is not consumed by a GPS function, the load on the worker in terms of battery replacement, can be reduced.

(Example of system configuration of estrus notifying system)

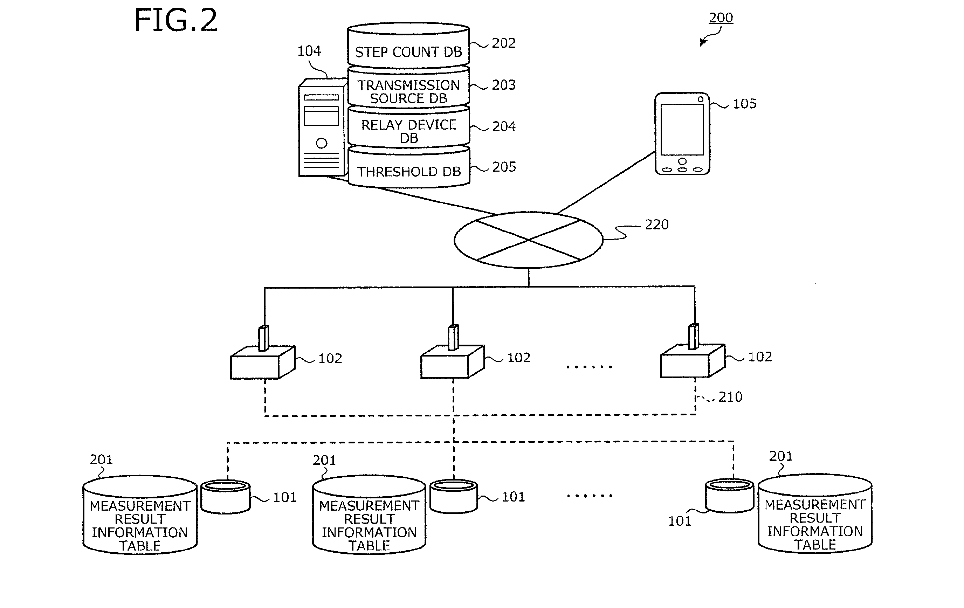

An example of system configuration of an estrus notifying system of the present embodiment will be described. FIG. 2 is a diagram depicting an example of system configuration of an estrus notifying system 200. In FIG. 2, although the estrus notifying system 200 is depicted to include the communications devices 101 in plural, the estrus notifying system 200 may include a single communications device 101.

As depicted in FIG. 2, in the case of an estrus notifying system having the communications device 101 in plural, each of the communications devices 101, when transmitting measurement result information, further transmits communications device identification information thereof. Here, the communications device identification information is information that can identify one communications device 101 from among plural communications devices 101. For example, the communications device identification information is information representing a communications device ID specific to each of the communications devices 101.

In the estrus notifying system 200, the communications device 101 and the relay device 102 are connected through a wireless communications network 210. The communications device 101 and the relay device 102 each have a given range around the device 101, 102 (e.g., a range of a 150-meter radius around the device 101, 102), as a communications area in which communication through the wireless communications network 210 is possible. When having a positional relation enabling communication, the communications device 101 and the relay device 102 are connected by the wireless communications network 210. For example, near field communication such as radio frequency identification (RFID) is applicable as the wireless communications network 210.

Further, the relay device 102, the estrus notifying apparatus 104, and the client apparatus 105 are connected via a network 220. For example, the network 220 is the Internet, a local area network (LAN), a wide area network (WAN), etc.

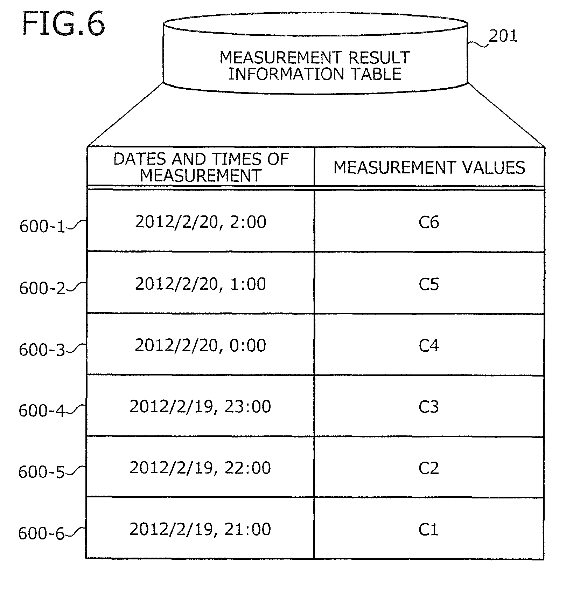

The communications device 101 has a measurement result information table 201 and is a portable computer attached to each livestock animal A raised on the farm F. The communications device 101 has a function of measuring the step count of the livestock animal A to which the communications device 101 is attached and a communications function through the wireless communications network 210. For example, a pedometer with an additional function of communication via the wireless communications network 210 is applicable as the communications device 101. Contents of the measurement result information table 201 will be described with reference to FIG. 6.

The relay device 102 is installed within the premises of the farm F and is a computer having a communications function via the wireless communications network 210 and communications function via the network 220. The relay devices 102 are respectively installed at a different installation site.

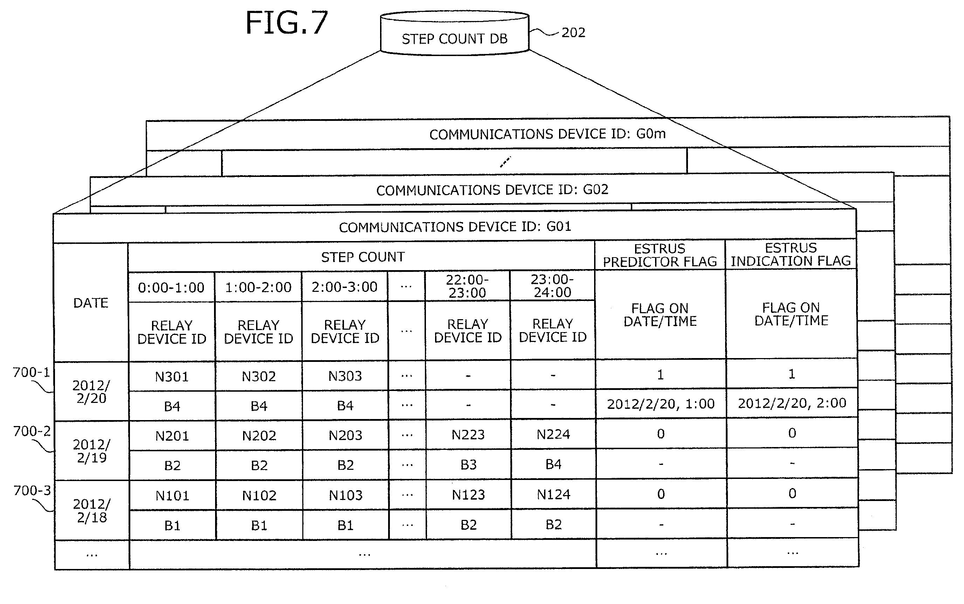

The estrus notifying apparatus 104 has a step count DB 202, a transmission source DB 203, a relay device DB 204, a threshold DB 205 and is a computer having a communications function via the network 220. For example, a server included in a cloud computing system, a personal computer (PC), note PC, etc. used by a manager of the farm F or the worker W are applicable as the estrus notifying apparatus 104. Contents of the step count DB 202, the transmission source DB 203, the relay device DB 204, and the threshold DB 205 will be described with reference to FIGs. 7 to 10.

The client apparatus 105 is a computer having the display 106 that displays images based on various types of information, and a communications function via the network 220. For example, a PC or note PC, mobile telephone, smartphone, and the like used by the worker W of the farm F are applicable as the client apparatus 105.

(Hardware configuration example of communications device)

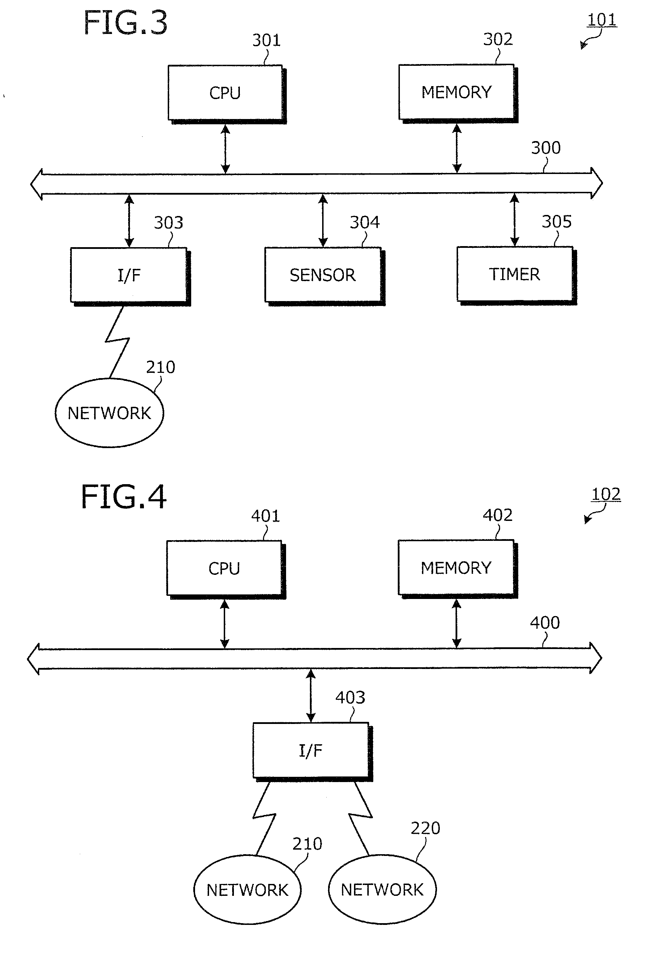

A hardware configuration example of the communications device 101 will be described. FIG. 3 is a block diagram depicting a hardware configuration example of the communications device of the present embodiment. In FIG. 3, the communications device 101 includes a central processing unit (CPU) 301, memory 302, an interface (I/F) 303, a sensor 304, and a timer 305, respectively connected by a bus 300.

Here, the CPU 301 governs overall control of the communications device 101. The memory 302 includes read-only memory (ROM), random access memory (RAM), and flash ROM. The ROM and the flash ROM, for example, store various types of programs such as a boot program. The RAM is used as a work area of the CPU 301.

The I/F 303 is connected to the wireless communications network 210 through a communications line and is connected to other apparatuses such as the relay devices 102, via the wireless communications network 210. The I/F 303 administers an internal interface with the wireless communications network 210 and controls the input and output of data from external apparatuses.

The sensor 304 outputs information for detecting behavior of the communications device 101. For example, when the sensor 304 is implemented by a gyroscope or a triaxial accelerometer and the communications device 101 accelerates, the sensor 304 outputs information according to the acceleration. The timer 305 has a function of measuring time. For example, the timer 305 is implemented by a real time clock (RTC) and measures the actual time. Further, the timer 305 may measure the time that elapses from a given time point. Configuration may be such that the timer 305 is disposed external to the communications device 101 and the communications device 101 obtains the measurement results of the timer 305 through the wireless communications network 210.

### (Hardware configuration example of relay device)

A hardware configuration example of the relay device 102 will be described. FIG. 4 is a block diagram depicting a hardware configuration example of the relay device 102. In FIG. 4, the relay device 102 includes a CPU 401, memory 402, and an I/F 403, respectively connected by a bus 400.

Here, the CPU 401 governs overall control of the relay device 102. The memory 402 includes ROM, RAM, and flash ROM. The ROM and flash ROM, for example, store various types of programs such as a boot program. The RAM is used as a work area of the CPU 401.

The I/F 403 is connected to the wireless communications network 210 through a communications line and is connected to other apparatuses such as the communications device 101, via the wireless communications network 210. The I/F 403 is further connected to the network 220 through a communications line and through the network 220, is connected to other apparatuses such as the estrus notifying apparatus 104. The I/F 403 administers an internal interface with the wireless communications network 210 and the network 220; and controls the input and output of data from external apparatuses.

### (Hardware configuration example of estrus notifying apparatus and client apparatus)

A hardware configuration example of the estrus notifying apparatus 104 and the client apparatus 105 will be described. Here, the estrus notifying apparatus 104 and the client apparatus 105 will be indicated as simply "the estrus notifying apparatus 104, etc.".

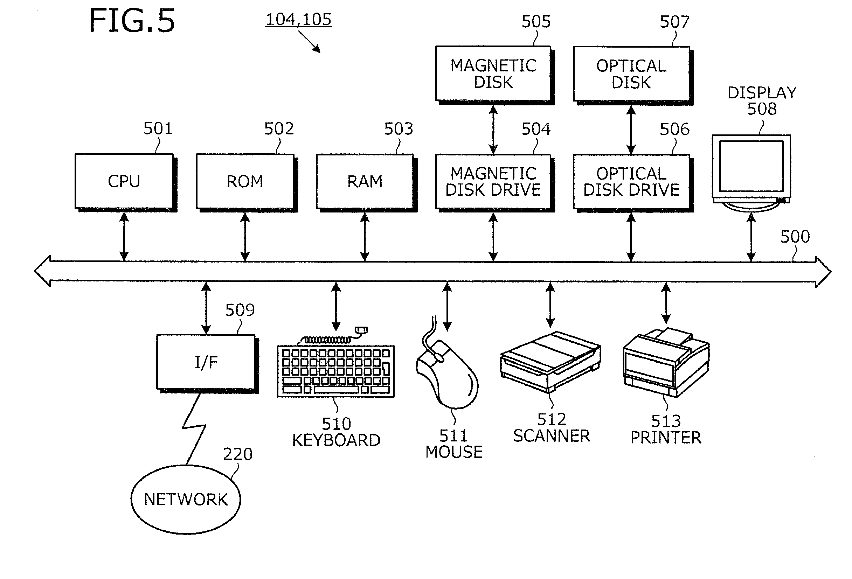

FIG. 5 is a block diagram depicting a hardware configuration example of the estrus notifying apparatus 104, etc. In FIG. 4, the estrus notifying apparatus 104, etc. include a CPU 501, ROM 502, RAM 503, a magnetic disk drive 504, a magnetic disk 505, an optical disk drive 506, an optical disk 507, a display 508, an I/F 509, a keyboard 510, a mouse 511, a scanner 512, and a printer 513, respectively connected by a bus 500.

Here, the CPU 501 governs overall control of the estrus notifying apparatus 104, etc. The ROM 502 stores programs such as a boot program. The RAM 503 is used as a work area of the CPU 501. The magnetic disk drive 504, under the control of the CPU 501, controls the reading and writing of data with respect to the magnetic disk 505. The magnetic disk 505 stores data written thereto under the control of the magnetic disk drive 504.

The optical disk drive 506, under the control of the CPU 501, controls the reading and writing of data with respect to the optical disk 507. The optical disk 507 stores data written thereto under the control of the optical disk drive 506, the data being read out by a computer.

The display 508 displays documents, images, and functional information in addition to a cursor, icons, and toolboxes. A CRT, TFT liquid crystal display, plasma display, and the like may be employed as the display 508.

The I/F 509 is connected to the network 220 through a communications line, and is connected to other apparatuses such as the relay device 102 and the client apparatus 105, via the network 220. The I/F 509 administers an internal interface with the network 220, and controls the input and output of data from external apparatuses. A modem, LAN adapter, etc. may be employed as the I/F 509.

The keyboard 510 has keys for inputting text, numerals, various instructions, etc. and inputs data. The keyboard 510 may be a touch panel input pad, a numeric pad, etc. The mouse 511 is used to move the cursor, select a range, move or change the size of a window, etc. As long as functions identical to a pointing device are provided, a trackball, a joy stick, and the like may be employed.

The scanner 512 optically reads images and takes in image data into the estrus notifying apparatus 104. The scanner 512 may have an optical character reader (OCR) function. The printer 513 prints image data and document data. A laser printer or ink jet printer may be employed as the printer 513.

Further, for example, among the components described above, the estrus notifying apparatus 104 may be configured to omit the optical disk drive 506, the optical disk 507, the display 508, the mouse 511, the scanner 512, and the printer 513. The client apparatus 105 may be configured to omit the optical disk drive 506, the optical disk 507, the mouse 511, the scanner 512, and the printer 513.

### (Example of information stored by communications device)

An example of the information stored by the communications device 101 will be described. As described above, the communications device 101 stores the measurement result information table 201. For example, the measurement result information table 201 is implemented by the memory 302 of the communications device 101.

### <Example of contents of measurement result information table>

FIG. 6 is a diagram depicting an example of the contents of the measurement result information table 201. In FIG. 6, the measurement result information table 201 has fields for dates and times of measurement, and measurement values. By setting information into these fields, measurement result information for each combination of a measurement value and the date and time of the measurement is stored as a record in the measurement result information table 201. For instance, in the example depicted in FIG. 6, records of measurement result information 600-1 to 600-6 are stored.

Here, the date and time of measurement represents the date and time of past transmissions of measurement result information. In the case of the present embodiment, as one example, the date and time of measurement indicates the date and time of the most recent six transmissions of the measurement result information. The measurement value represents a past measurement value of the step count of the livestock animal A at the time of transmission of the measurement result information. In the case of the present embodiment, as one example, the measurement values of the step counts of the livestock animal A at the time of the most recent six transmissions of the measurement result information are indicated.

For example, the communications device 101 cumulates and stores as the current measurement value, the step count of the livestock animal A from the time when the measurement value is set to "0" until the current time. With each step that the livestock animal A takes, the communications device 101 instantaneously accelerates. Upon detecting this acceleration via the sensor 304, the communications device 101 increments the current measurement value by "+1".

When the transmission time for the measurement result information arrives according to the measurement result of the timer 305, the communications device 101 stores measurement result information in which the current measurement value is correlated with the date and time of measurement for this transmission time. If the transmission time for the measurement result information is a one-hour interval, for example, transmission occurs every hour, on the hour.

For example, in FIG. 6, the measurement result information 600-1 indicates that the measurement value at 2:00 on 2012/02/20 is "C6". Here, "C6" is a positive integer. Upon storing the measurement result information to the measurement result information table 201, the communications device 101 transmits to the estrus notifying apparatus 104, via the relay device 102, each record of measurement result information stored in the measurement result information table 201. In the example depicted in FIG. 6, the communications device 101 transmits the measurement result information 600-1 to 600-6.

As a result, the communications device 101 is able to transmit measurement result information multiple times. For example, the measurement result information 600-1 is transmitted six times, at one-hour intervals from 21:00 on 2112/02/19 until 2:00 on 2012/02/20. Therefore, even if the communications device 101 cannot communicate with any of the relay devices at the current transmission time for the measurement result information and the transmission of the measurement result information fails, the communications device 101 can transmit at a subsequent transmission time, the measurement result information for which transmission failed.

In this example, although an example is described where the communications device 101 stores measurement result information for the six most recent measurements, configuration is not limited hereto. Configuration may be such that the communications device 101 does not store past measurement result information. For example, in this case, when the transmission time for the measurement result information arrives, the communications device 101 may transmit the current measurement value as the measurement result information and delete the measurement result information. With such a configuration, in terms of storage of the measurement result information, the volume of data that the communications device 101 has to store can be reduced.

Although the communications device 101 is described to transmit each record of measurement result information stored in the measurement result information table 201, configuration is not limited hereto. For example, configuration may be such that the communications device 101 transmits only the one most recent record of measurement result information. More specifically, in the case of the example depicted in FIG. 6, the communications device 101 may be configured to transmit only the measurement result information 600-1. In this case, in terms of the transmission of the measurement result information, the volume of data that the communications device 101 has to transmit can be reduced.

Furthermore, configuration may be such that when the communications device 101 transmits the one most recent record of measurement result information, the communications device 101 also transmits the measurement result information for which transmission failed at a past transmission time. For example, in this case, upon receiving measurement result information from the communications device 101, the relay device 102 transmits to the communications device 101, successful reception information indicating that measurement result information has been received. If the communications device 101 does not receive the successful reception information within a given period from the transmission of the measurement result information, the communications device 101 determines that this transmission of the measurement result information has failed.

In this case, the communications device 101 correlates and stores the measurement result information for which transmission failed and information indicating that the transmission failed. Thereafter, when the transmission time for the measurement result information arrives, the communications device 101 transmits the one most recent record of the measurement result information and the measurement result information for which transmission failed. With such a configuration, in terms of transmission of the measurement result information, the volume of data that the communications device 101 has to transmit can be reduced while assuredly transmitting measurement result information to the relay device 102.

### (Example of information stored by estrus notifying apparatus)

An example of information stored by the estrus notifying apparatus 104 will be described. First, examples of the contents of the various DBs 202, 203, 204, and 205 stored by the estrus notifying apparatus 104 will be described. For example, the various DBs, 202, 203, 204, and 205 described hereinafter are implemented by a memory apparatus such as the ROM 502, the RAM 503, the magnetic disk 505, and the optical disk 507 of the estrus notifying apparatus 104.

### <Example of contents of step count DB>

FIG. 7 is a diagram depicting an example of the contents of the step count DB 202. In FIG. 7, the step count DB 202 has fields for dates, step counts, estrus predictor flags, and estrus indication flags. By setting information into these fields, step count information for each combination of a date, step count, estrus predictor flag, and estrus indication flag is stored as a record in the step count DB 202. In the example depicted in FIG. 7, records of step count information 700-1 to 700-3 are stored in the step count DB 202.

Here, the date is the date that the step count was measured and is indicated as YEAR/MONTH/DAY, for example. The step count indicates the step count of the livestock animal A, based on count result information. The step count field includes, for example, time slot fields such as "0:00-1:00", "1:00-2:00", "2:00-3:00", ..., "22:00-23:00", and "23:00-24:00". Each of the time slot fields stores information indicating the step count of the livestock animal A for the respective time slot and the identification information of the relay device that transmitted the information. For example, the estrus notifying apparatus 104 stores into each time slot field, as the step count of the livestock animal A, the difference of the measurement value at the last time less the measurement value at the head time.

More specifically, for example, assuming that the measurement value at 2:00 on 2012/02/20 is "C6" and the measurement value at 1:00 on 2012/02/20 is "C5". As described above, "C6" and "C5" are positive integers. In this case, as the step count of the livestock animal A, the estrus notifying apparatus 104 stores into the "1:00-2:00" time slot field for the date "2012/02/20", "N302(N302=C6-C5)", which is the difference of "C6" less "C5". Further, "B4" is stored as the identification information of the relay device that transmitted the information received.

The estrus predictor flags and the estrus indication flags include an ON/OFF field that indicates whether the flag is "ON" or "OFF". In the case of the present embodiment, as one example, if a flag is ON, "1" is set in the ON/OFF field. If a flag is "OFF", "0" is set in the ON/OFF field. The estrus predictor flags and the estrus indication flags further include, for example, a flag ON date/time field storing the date and time when the flag was set to "ON".

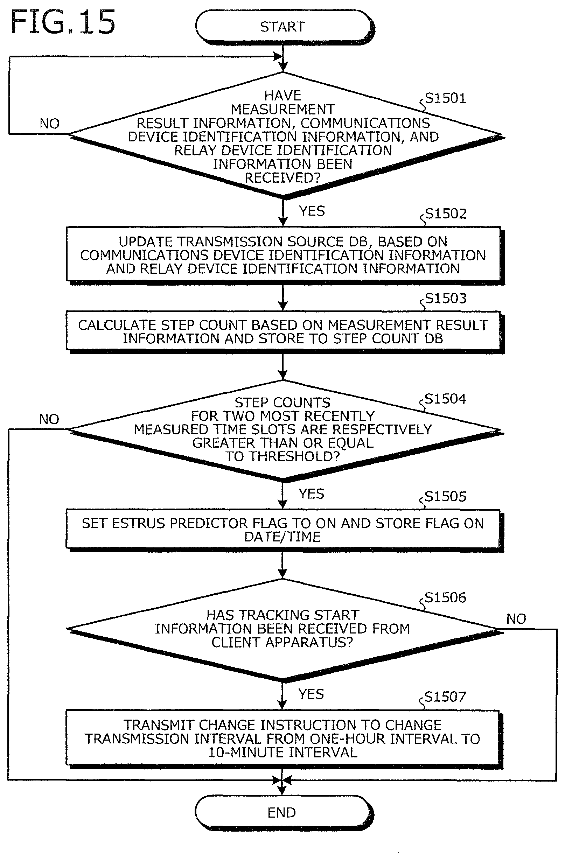

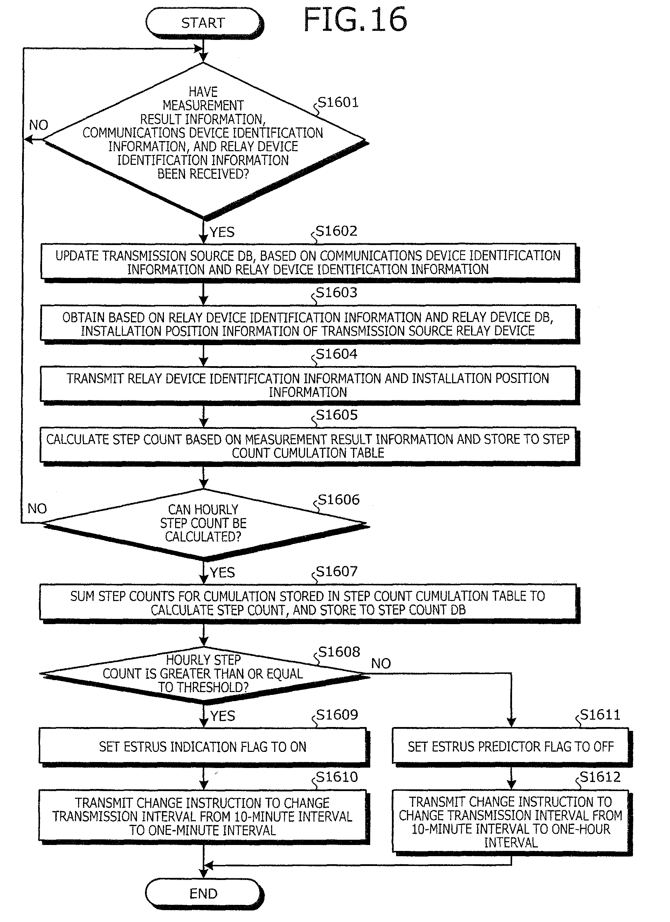

For example, when a given condition is satisfied, the estrus notifying apparatus 104 stores "1" into the ON/OFF field of an estrus predictor flag or an estrus indication flag. On the other hand, when "1" is not stored in the ON/OFF field, the estrus notifying apparatus 104 stores "0". The setting of the estrus predictor flags and the estrus indication flags will be described with reference to FIGs. 15 and 16 hereinafter.

In the step count DB 202, each of the above fields is set for each of the communications devices 101. For example, in FIG. 7, fields of the step count DB 202, for the communications device 101 of the communications device ID "G01" are depicted. In the step count DB 202, the estrus notifying apparatus 104 stores step count information and relay device identification information for each of the communications devices 101 and based on the stored step count information, determines whether the step count satisfies a given condition.

More specifically, for example, the communications device 101 transmits the communications device identification information thereof when transmitting measurement result information. The estrus notifying apparatus 104 receives the measurement result information and the communications device identification information. Each of the communications devices 101 have a communications device ID, and from the received communications device identification information, the estrus notifying apparatus 104 identifies the communications device 101 that transmitted the measurement result information. The estrus notifying apparatus 104 calculates and stores into the fields of the step count DB 202, for the identified communications device ID, the step count based on the identified communications device ID received together with the measurement result information. The estrus notifying apparatus 104 determines whether the stored step count satisfies a given condition.

### <Example of contents of transmission source DB>

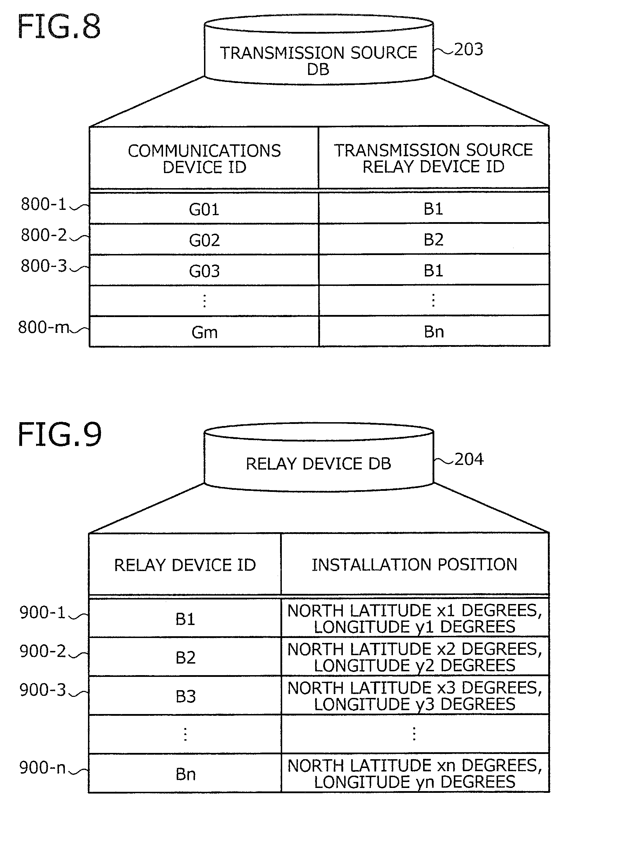

An example of the contents of the transmission source DB 203 will be described. FIG. 8 is a diagram depicting an example of the contents of the transmission source DB 203. In FIG. 8, the transmission source DB 203 has fields for communications device IDs and transmission source relay device IDs. By setting information into these fields, transmission source relay device information for each combination of communications device ID and transmission source relay device ID is stored as records in the transmission source DB 203. For instance, in the example depicted in FIG. 8, records of transmission source relay device information 800-1 to 800-m are stored in the transmission source DB 203.

A communications device ID is an identifier of a communications device 101. A transmission source relay device ID is an identifier of a transmission source relay device 102 that transmitted to the estrus notifying apparatus 104, the measurement result information just transmitted by the communications device 101. For example, in FIG. 8, transmission source relay device information 800-1 indicates that the measurement result information just transmitted by the communications device 101 of the communications device ID "G01" has been transmitted to the estrus notifying apparatus 104, via the relay device 102 of the relay device ID "B1".

In the case of the present embodiment, the estrus notifying apparatus 104 stores by the transmission source DB 203, information indicating the relay device 102 through which the measurement result information just transmitted by a communications device 101 is received. Thus, for example, when the worker W requests the output of transmission source relay device information for a given communications device 101, the estrus notifying apparatus 104 can output this transmission source relay device information to the client apparatus 105, etc. As a result, when a desired communications device 101 transmits recent measurement result information, the worker W can know in which relay device 102 communications area, the desired communications device 101 is located.

### <Contents of relay device DB>

An example of the contents of the relay device DB 204 will be described. FIG. 9 is a diagram depicting an example of the contents of the relay device DB 204. In FIG. 9, the relay device DB 204 has fields for relay device IDs and installation positions. By setting information into these fields, installation position information for each combination of a relay device ID and an installation position is stored as a record in the relay device DB 204. For instance, in the example depicted in FIG. 9, records of installation position information 900-1 to 900-n are stored in the relay device DB 204.

Here, a relay device ID is an identifier of a relay device 102. An installation position is installation position information indicating the installation position of a relay device 102. Installation position information is information that can specify one geographical point on a map and, for example, is information representing latitude and longitude, information representing coordinates, and the like. For example, in FIG. 9, installation position information 900-1 indicates that the relay device 102 of the relay device ID "B1" is installed at a geographical point indicated by a "north latitude of x1 degrees and longitude of y1 degrees".

### <Contents of threshold DB>

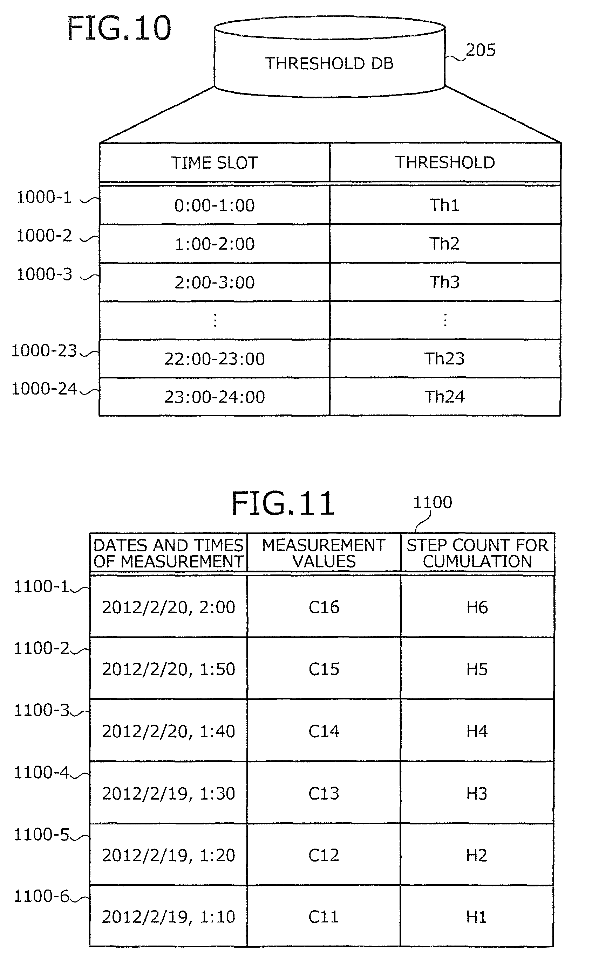

An example of the contents of the threshold DB 205 will be described. FIG. 10 is a diagram depicting an example of the contents of the threshold DB 205. In FIG. 10, the threshold DB 205 has fields for time slots and thresholds. By setting information into these fields, threshold information for each combination of a time slot and a threshold is stored as a record in the threshold DB 20. For instance, in the example depicted in FIG. 10, records of threshold information 1000-1 to 1000-24 are stored in the threshold DB 205.

A time slot is information for specifying whether a threshold for a step count stored in a time slot field of the step count DB 202 is to be used. The time slots are provided corresponding to the time slot fields of the step count DB 202. A threshold represents a threshold for determining whether the livestock animal A to which the communications device 101 is attached is in estrus. For example, the estrus notifying apparatus 104 determines if the step count stored in the time slot field "0:00-1:00" in the step count DB 202 is greater than or equal to a threshold "Th1" for a case where the time slot is "0:00-1:00".

Each of the thresholds Th1 to Th24 may be a different value or may be the same value. For example, if the thresholds Th1 to Th24 that take into consideration the biology of the livestock animal A are defined, the effects of the biology of the livestock animal A can be reduced and the accuracy in detecting estrus of the livestock animal A can be increased. More specifically, for example, if the livestock animal A is a nocturnal animal, the threshold for 22:00 to 3:00 is set to be higher than the thresholds for other time slots.

Further, the thresholds Th1 to Th24 may be values that differ according to the livestock animal A or may be a common value for each livestock animal A. For example, by setting for each livestock animal A, the thresholds Th1 to Th24 based on a history of step counts obtained in the past, the effects of individual differences of the livestock animals A can be reduced and the accuracy in detecting estrus of the livestock animals A can be increased. More specifically, for example, the threshold for a livestock animal A that actively walks on a regular basis can be set higher than the thresholds for other livestock animals A.

As described, in the case of the present embodiment, the thresholds Th1 to Th24 of the threshold DB 205 are defined for hourly step counts. Thus, if measurement result information is transmitted to the estrus notifying apparatus 104 at 10-minute intervals, the estrus notifying apparatus 104 sums six of the 10-minute step counts to calculate an hourly step count and compares the calculated hourly step count and the threshold in the threshold DB 205.

### <Example of contents of step count cumulation table>

FIG. 11 is a diagram depicting an example of the contents of a step count cumulation table 1100. The estrus notifying apparatus 104 stores the step count cumulation table 1100 depicted in FIG. 11. For example, the step count cumulation table 1100 is stored in a memory apparatus such as the RAM 503, the magnetic disk 505, and the optical disk 507 of the estrus notifying apparatus 104.

In FIG. 11, a step count cumulation table 1100 has fields for dates and times of measurement, measurement values, and step counts for cumulation. By setting information into these fields, step-count-for-cumulation information for each combination of a date and time of measurement, a measurement value, and a step count for cumulation is stored as a record in the step count cumulation table 1100. In the example depicted in FIG. 11, records of step-count-for-cumulation information 1100-1 to 1100-6 are stored.

Here, a date and time of measurement represents a date and time indicated by received measurement result information. A measurement value represents a measurement value indicated by the received measurement result information. A step count for cumulation represents a 10-minute step count of a livestock animal A. The step count for cumulation can be obtained by subtracting a previous measurement value among two consecutive measurement values in the step count cumulation table 1100 from the subsequent measurement value among the two consecutive measurement values.

For example, as depicted in FIG. 11, the estrus notifying apparatus 104 is assumed to receive measurement result information for dates and times of measurement including "1:10", "1:20", ..., "1:50", and "2:00". In this example, the dates of the measurement result information are the same date. In the example depicted in FIG. 11, the date is "2012/02/20". Further, measurement values indicated by the measurement result information are "C11", "C12", ..., "C15", and "C16".

In this case, for example, the step count for cumulation for the date and time of measurement "2:00" is "H6", which is the difference of "C16" less the measurement value "C15" of the previous date and time of measurement "1:50", or more specifically, H6=C16-C15. The step count for cumulation for the date and time of measurement "1:50" is "H5", which is the difference of "C15" less the measurement value "C14" of the previous date and time of measurement "1:40", or more specifically, H5=C15-C14.

The estrus notifying apparatus 104 stores to the time slot field "1:00-2:00" for the date "2012/02/20" the value obtained by integrating H1 to H6. In the example depicted in FIG. 7, "N302" is stored in the time slot field "1:00-2:00" for the date "2012/02/20". For example, the value of "N302" in FIG. 7 is the sum of "H1 to H6" in FIG. 11.

### (Functional configuration example of estrus notifying apparatus 104)

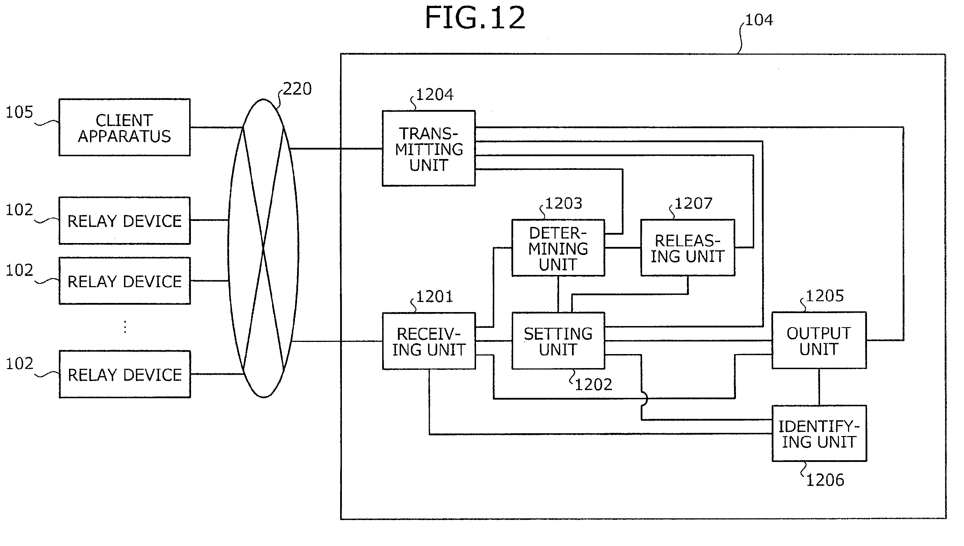

FIG. 12 is a block diagram depicting a functional configuration example of the estrus notifying apparatus 104. In FIG. 12, the estrus notifying apparatus 104 includes a receiving unit 1201, a setting unit 1202, a determining unit 1203, a transmitting unit 1204, an output unit 1205, an identifying unit 1206, and a releasing unit 1207. These functions forming a control unit, for example, the receiving unit 1201 to the releasing unit 1207, are implemented by executing on the CPU 501, a program stored on the magnetic disk 505 depicted in FIG. 5 or by the I/F 509, the magnetic disk 505, etc. Process results of each functional unit are stored to the RAM 503, for example.

The receiving unit 1201 has a function of receiving via the network 220, measurement result information indicating measurement results of the step count of the livestock animal A, from the communications device attached to the livestock animal A in the communications area of a relay device 102 among the relay devices 102 at different installation positions. Further, the receiving unit 1201 receives communications device identification information of the communications device 101 and the relay device identification information of the relay device 102 used to report the measurement result information. As described above, for example, the communications device identification information is information indicating the communications device ID and the relay device identification information is information indicating the relay device ID.

For example, the relay device 10 receives measurement result information from the communications device 1012, at the first transmission interval (e.g., one-hour interval), the second transmission interval (e.g., 10-minute interval), or a third transmission interval (e.g., one-minute interval). Each time measurement result information is received, the relay device 102 transmits the received measurement result information to the estrus notifying apparatus 104.

The setting unit 1202 has a function of setting the communications device 101 that transmitted the measurement result information to be a tracking target. For example, the setting unit 1202, in a step count DB 202, sets an estrus predictor flag or an estrus indication flag of the step count information to ON to thereby, set the communications device 101 having the communications device ID correlated with the step count information to be a tracking target. More specifically, for example, the setting unit 1202 may set the communications device 101 to be a tracking target, based on a determination result of the determining unit 1203 described hereinafter.

The determining unit 1203 has a function of determining based on the measurement result information received by the receiving unit 1201, whether the step count of the livestock animal A satisfies a given condition. The given condition, for example, can be arbitrarily set according to the abnormal state that the worker W wants to find such as estrus, illness, etc. of the livestock animals A. For example, as with the present embodiment, in a case where a female cow in estrus is to be found, the characteristic that the step count of female cows increases when in estrus is used and the given condition is set to be a condition that the step count of the livestock animal be greater than or equal to a given threshold. Further, when an ill livestock animal A is to be found, the characteristic that the step count of the livestock animal A decreases consequent to illness is used and the given condition is set to be a condition that the step count of the livestock animal be less than a separately determined threshold.

Further, for example, for transient abnormal states such as when a predator appears, from the view point of preventing the communications device 101 from being set to be a tracking target, the determining unit 1203 may determine whether each step count for sequentially measured given periods satisfies a given condition. In this case, the determining unit 1203 performs the determination based on the differing step counts of measured time slots. In the case of the present embodiment, as one example, the determining unit 1203 determines whether the two most recently measured, consecutive one-hour step counts stored in the step count DB 202 are respectively greater than or equal to a threshold defined by the threshold DB 205.

Further, if the given condition is satisfied, such as when a livestock animal A has been determined to be in estrus, the determining unit 1203 notifies the worker W of the communications device ID of the communications device 101 attached to the livestock animal A, and the relay device ID and installation position information of the relay device 102 used to report the measurement result information of the livestock animal A. More specifically, for example, the determining unit 1203 controls the transmitting unit 1204 to transmit to the client apparatus 105, the communications device ID of the communications device 101, and the relay device ID and installation position information of the relay device 102 and thereby, notifies the worker W.

Further, when notification of attending to the livestock animal A is received from the worker W, the setting unit 1202 may set the communications device 101 attached to the livestock animal A to be a tracking target. More specifically, for example, if tracking start information is received from the client apparatus 105 by the receiving unit 1201, the setting unit 1202 sets the communications device 101 attached to the livestock animal A to be a tracking target. The tracking start information is information giving notification that the worker W will attend to the livestock animal A.

Thus, when estrus of a livestock animal A is detected and notification indicating that the livestock animal will be attended to is received from the worker, the setting unit 1202 can set the communications device 101 attached to the livestock animal A to be a tracking target. If a given condition is determined to be satisfied by the determining unit 1203, the setting unit 1202 may set the communications device 101 attached to the livestock animal A to be a tracking target.

Further, if measurement result information transmitted at the second transmission interval is received by the receiving unit 1201, the determining unit 1203 may determine based on the measurement result information transmitted at the second transmission interval, whether to change the communication interval of the communications device 101 to the third transmission interval, which is shorter than the second transmission interval. For example, in the present embodiment, a threshold for a one-hour step count is defined. Therefore, as depicted in FIG. 11, the determining unit 1203 stores by the step count cumulation table 1100, measurement values indicated by measurement result information for six transmissions at 10-minute intervals. The determining unit 1203 determines if a value obtained by cumulating the measurement values for six transmissions is greater than or equal to a threshold defined by the threshold DB 205.

Thus, the determining unit 1203 can make the determination above even without preparing separate thresholds for each step count measured for periods of differing lengths and can reduce the data volume for storing the thresholds. Here, although the determining unit 1203 is described to cumulate step counts for each 10-minute interval for comparison to a threshold, configuration is not limited hereto. For example, if the step counts for each 10-minute interval exhibit a trend of increasing, the determining unit 1203 may determine to use the third transmission interval. More specifically, for example, in this case, for H1<H2, H2<H3 in the example depicted in FIG. 11, the determining unit 1203 determines to use the third transmission interval.

On the contrary, if the step intervals for each 10-minute interval exhibit a trend of decreasing, the determining unit 1203 may determine to use the third transmission interval. More specifically, for example, in this case, for H3<H2, H2<H1 in the example depicted in FIG. 11, the determining unit 1203 determines to use the third transmission interval.

The transmitting unit 1204 has a function of transmitting to the communications device 101, via the relay device 102, a change instruction to change the transmission interval of the communications device 101 for transmitting the measurement results of step counts of the livestock animal A to the second transmission interval, which is shorter than the first transmission interval, if the communications device 101 has been set to be a tracking target by the setting unit 1202. For example, if the estrus predictor flag has been set to ON by the setting unit 1202, the transmitting unit 1204 transmits a change instruction to change the transmission interval to the second transmission interval, which includes information indicating the communications device ID correlated to the step count information for which the estrus predictor flag was set to ON.

For example, the transmitting unit 1204 transmits a change instruction to all of the relay devices 102. Each of the relay devices 102 transmits the change instruction to all communications device 101 in the communications area thereof. Upon receiving the change instruction from the relay devices 102, each of the communications devices 101 determines whether the communications device ID thereof is included in the change instruction and if the communications device ID thereof is included, the communications device 101 sets the transmission interval according to the change instruction.

Thus, if the communications device 101 is located at a position enabling communication with any one of the relay devices 102, the communications device 101 can receive the change instruction and set the transmission interval. In other words, the communications device 101 can receive the change instruction from a relay device 102 different from the relay device 102 that transmitted the most recent measurement result information and can change the transmission interval according to the change instruction.

Further, if the determining unit 1203 has determined to change the transmission interval to the third transmission interval, the transmitting unit 1204 may transmit to the communications device 101, via a relay device 102, a change instruction to change the transmission interval of the communications device 101 for transmitting measurement results of step counts of the livestock animal A, to the second transmission interval, which is shorter than the first transmission interval.

The output unit 1205 outputs the identification information of the relay device 102 that transmitted the measurement result information from the communications device 101 set to a tracking target. More specifically, for example, the output unit 1205 may output the identification information of the transmission source relay device 102, each time a measurement result is received from the communications device 101 set as a tracking target. The identification information of the relay device 102 is information enabling identification of one relay device 102 among the relay devices 102 and in the present embodiment, is assumed to be the relay device ID.

Further, the output unit 1205 may output the installation position information identified by the identifying unit 1206. Here, each time a measurement result of the step count for the livestock animal A is received from the communications device 101 set to be a tracking target, the identifying unit 1206 refers to a memory unit storing installation position information that indicates the installation positions of each of the relay devices 102. The identifying unit 1206 has a function of identifying the installation position information of the relay device 102 that transmitted the received measurement result. In the case of the present embodiment, as one example, the identifying unit 1206 identifies in the relay device DB 204 and based on the relay device ID of the relay device 102 set to be a tracking target, the installation position information correlated with the relay device ID. The output unit 1205 outputs the installation position information identified by the identifying unit 1206.

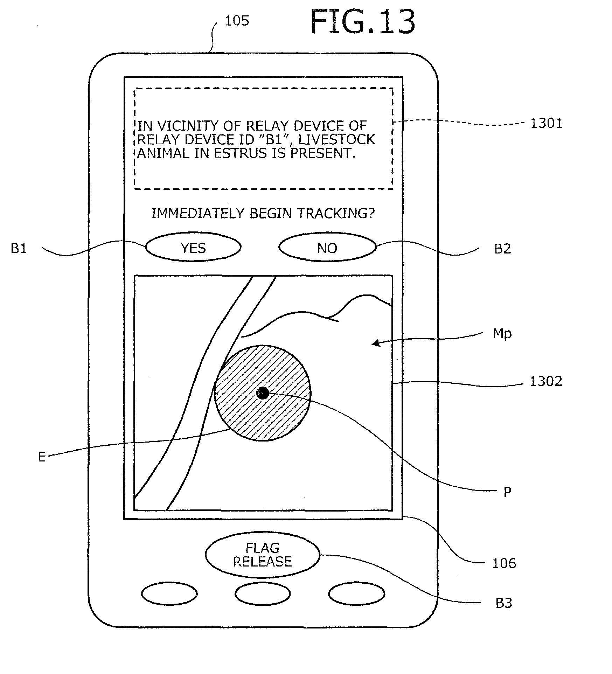

For example, the output unit 1205 outputs relay device identification information and installation position information to the transmitting unit 1204, whereby the relay device identification information and installation position information are transmitted to the client apparatus 105 by the transmitting unit 1204, via the network 220. Thus, the client apparatus 105 can display an image based on the received relay device identification information and installation position information. A concrete display example of the client apparatus 105 will be described hereinafter with reference to FIG. 13. Further, the output unit 1205 may output the relay device identification information and installation position information by displaying a given image on the display 508 of the estrus notifying apparatus 104.

The output unit 1205 notifies the worker W of the installation position information of the new relay device 102, each time the relay device 102 used to report the measurement result information from the communications device 101 set to be a tracking target, changes. More specifically, for example, the output unit 1205 refers to the step count DB 202 or the transmission source DB 203 and determines whether the relay device ID of the relay device 102 used this time to report measurement result information and the relay device ID of the relay device 102 used the previous time coincide. If the relay device IDs do not coincide, the output unit 120, via the network 220, notifies the client apparatus 105 of the installation position information of the new relay device 102.

The releasing unit 1207 has a function of releasing the tracking target setting of the communications device 101, if the determining unit 1203 determines to not change to the third transmission interval. For example, the releasing unit 1207 sets the estrus predictor flag to OFF in the step count DB 202 to thereby, release the tracking target setting of the communications device 101 having the communications device ID correlated with the step count information. If the tracking target setting of the communications device 101 has been released by the releasing unit 1207, the transmitting unit 1204 transmits to the communications device 101, via a relay device 102, a change instruction to change the transmission interval to the first transmission interval. Thus, the estrus notifying apparatus 104 can change the communication interval of the communications device 101 to the first communication interval and can suppress the battery consumption of the communications device 101.

### (Display example of client apparatus)

A display example of the client apparatus 105 will be described. The display example to be described, for example, is displayed on the display 106, if the client apparatus 105 has received relay device identification information and installation position information from the estrus notifying apparatus 104.

FIG. 13 is a diagram depicting an example of display by the client apparatus 105. In FIG. 13, for example, on the display 106, a message 1301 pointing the worker W to the relay device 102 identified by the relay device identification information is displayed. For example, here, the relay device identification information is assumed to indicate the relay device 102 of the relay device ID "B1". In this case, as depicted in FIG. 13, the client apparatus 105 displays a message 1301, "Livestock animal in estrus is near relay device of relay device ID 'B1'" on the display 106.

The worker W, knowing where the relay device 102 having the relay device ID pointed to by the message 1301 is installed, can surmise from the message 1301 where the livestock animal is that has the communications device 101 being tracked. Therefore, when searching for the livestock animal having the communications device 101 being tracked, the worker W can narrow down the range to be searched, enabling the work load and time consumed for searching for the livestock animal A to be reduced.

Further, an installation position image 1302 pointing the worker W to a geographical point specified by installation position information is displayed on the display 106. Further, buttons B1, B2 for indicating whether tracking is to be started immediately and a flag release button B3 are displayed. For example, if button B1 is pressed by the worker W, the client apparatus 105 transmits to the estrus notifying apparatus 104, tracking start information representing notification that tracking of the livestock animal A is to be started. By receiving the tracking start information, the estrus notifying apparatus 104 can determine that the worker W has started the tracking of the livestock animal A.

Further, if button B2 is pressed by the worker W, the client apparatus 105 transmits to the estrus notifying apparatus 104, no-tracking information representing notification that tracking of the livestock animal A is not to be started. By receiving the no-tracking information, the estrus notifying apparatus 104 can determine that the worker W will not track the livestock animal A.

Further, if button B3 is pressed by the worker W, the client apparatus 105 transmits to the estrus notifying apparatus 104, a flag release request for setting the estrus indication flag for the livestock animal A to OFF. By receiving the flag release request, the estrus notifying apparatus 104 can set the estrus indication flag of the livestock animal A to OFF.

Here, the installation position image 1302 includes the map image Mp and the geographical point image P. The map image Mp is an image that represents a given map range that includes the geographical point specified by the installation position information. The geographical point image P is an image that represents on the map image Mp, the geographical point specified by the installation position information. As depicted in FIG. 13, the installation position image 1302 is an image in which the geographical point image P is displayed superimposed on the map image Mp.

The client apparatus 105 may further display the communications area image E, which represents the communications area of the relay device 102. Here, for example, the communications area image E is an image that represents a given range that includes the geographical point specified by the installation position information. The communications area image E does not have to strictly represent the communications area of the relay device 102. For example, the communications area image E may be an image that represents a 150-meter radius around the geographical point specified by the installation position information.

For example, if the client apparatus 105 stores map data, the client apparatus 105 generates based on the stored map data and the received installation information, image data for displaying the installation position image 1302. Further, if the estrus notifying apparatus 104 stores map data, the estrus notifying apparatus 104 may generate based on the stored map data and the installation position information, an image for displaying the installation position image 1302 and may transmit the generated image data to the client apparatus 105.

From the installation position image 1402, even an inexperienced worker W unable to determine the position of a relay device from the relay device ID can surmise the location of the livestock animal A having the traced communications device 101. Therefore, the worker W can narrow down the range to be searched for the livestock animal A having the traced communications device 101, enabling reductions in the work load and time consumed in searching for the livestock animal A.

### (Process procedure of communications device)

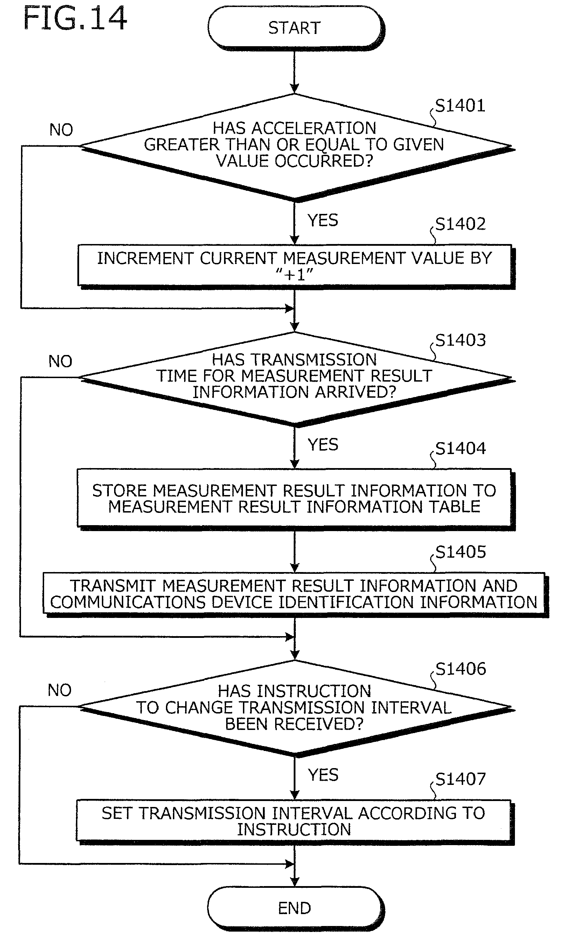

A process procedure of the communications device 101 will be described. FIG. 14 is a flowchart depicting an example of a process procedure of the communications device 101. In the flowchart depicted in FIG. 14, the communications device 101, via the sensor 304, determines whether acceleration of the communications device 101 has occurred that is greater than or equal to a given value (step S1401). If acceleration greater than or equal to a given value has not occurred (step S1401: NO), the communications device 101 transitions to the operation at step S1403.

If acceleration greater than or equal to a given value has occurred (step S1401: YES), the communications device 101 increments the current measurement value by "+1" (step S1402). Thus, each time acceleration of the communications device 101 occurs consequent to the livestock animal A walking, the communications device 101 can cumulate the current measurement value by +1. The communications device 101 determines whether the transmission time for the measurement result information has arrived (step S1403). For example, as described above, when the time measured by the timer 305 becomes a given time, the communications device 101 determines that the transmission time for the measurement result information has arrived.

At step S1403, the communications device 101 uses a different condition for each set transmission interval to determine the transmission time for the measurement result information. More specifically, if the transmission interval is set to a one-hour interval, the communications device 101 determines that the transmission time has arrived at given times of one-hour intervals, such as when the time measured by the timer 305 becomes "0:00", "1:00", ...

On the other hand, if the transmission interval is set to be a 10-minute interval, the communications device 101 determines that the transmission time for the measurement result information has arrived at given times of 10-minute intervals, such as when the time measured by the timer 305 becomes "0:10", "0:20", ... Further, if the transmission interval is set to be a one-minute interval, the communications device 101 determines that the transmission time for the measurement result information has arrived at given times of one-minute intervals, such as when the time measured by the timer 305 becomes "0:01", "0:02", ....

If the transmission time for the measurement result information has arrived (step S1403: YES), the communications device 101 stores to the measurement result information table 201, measurement result information in which the date and time of the current transmission time for the measurement result information and the current measurement value are correlated (step S1404). Thus, each time the transmission time arrives for measurement result information, the communications device 101 can store the measurement value for the corresponding time.

The communications device 101 transmits to the relay device 102, all measurement result information stored in the measurement result information table 201 and the communications device identification information thereof (step S1405), and transitions to step S1406. Thus, the communications device 101 can transmit measurement result information at a given transmission interval.

As described above, upon receiving measurement result information and communications device identification information from the communications device 101, the relay device 102 transmits to the estrus notifying apparatus 104, via the network 220, the received measurement result information and communications device identification information as well as the relay device identification information thereof.

At step S1403, if the transmission time has not arrived (step S1403: NO), the communications device 101 transitions to step S1406. The communications device 101 determines whether an instruction to change the transmission interval for the measurement result information has been received from the relay device 102 (step S1406). If no instruction to change the transmission interval has been received (step S1406: NO), the communications device 101 ends the series of operations according to the present flowchart.

If an instruction to change the transmission interval has been received (step S1406: YES), the communications device 101 sets the transmission interval according to the received instruction (step S1407), and ends the series of operations according to the present flowchart. Thus, the communications device 101 can change the transmission interval according to a received change instruction.

The communications device 101 may further reset the measurement value to "0" at a given timing. For example, when a given time has arrived (e.g., "0:00", every day), the communications device 101 resets the measurement value to "0". Further, the communications device 101 may reset the measurement value "0", if an instruction to reset the measurement value to "0" is received from the estrus notifying apparatus 104, via the relay device 102.

### (Process procedure of estrus notifying apparatus)

A process procedure of the estrus notifying apparatus 104 will be described. FIG. 15 is a flowchart (part 1) depicting an example of a process procedure of the estrus notifying apparatus 104. For example, the estrus notifying apparatus 104 performs the operations in the flowchart depicted in FIG. 15, if in the step count DB 202, no estrus predictor flag and no estrus indication flag is set to "1", i.e., ON.

In the flowchart depicted in FIG. 15, the estrus notifying apparatus 104 determines whether measurement result information, communications device identification information, and relay device identification information have been received from the relay device 102 (step S1501). The estrus notifying apparatus 104 stands by until measurement result information, communications device identification information, and relay device identification information are received (step S1501: NO).

Upon receiving measurement result information, communications device identification information, and relay device identification information (step S1501: YES), the estrus notifying apparatus 104 updates the stored contents of the transmission source DB 203, based on the received communications device identification information and relay device identification information (step S1502). For example, at step S1602, the estrus notifying apparatus 104 stores to the transmission source DB 203, transmission source relay device information in which the communications device ID specified by the received communications device identification information and the relay device ID specified by the received relay device identification information are correlated.

The estrus notifying apparatus 104 calculates the step count of the livestock animal A, based on the communications device identification information and the measurement result information received at step S1501 and stores the calculated step count to the step count DB 202 (step S1503). As described above, at step S1503, the estrus notifying apparatus 104 calculates a step count for each communications device 101, based on the received communications device identification information and measurement result information, and stores the calculated step counts. Calculation of the livestock animal A step counts, based on the received measurement result information has been described with reference to FIG. 6, etc. and therefore, description thereof will be omitted hereinafter.

The estrus notifying apparatus 104 determines whether among the step counts stored in the step count DB 202 for the communications device identification information received at step S1601, the step counts for the two most recently measured time slots are respectively greater than or equal to a threshold defined in the threshold DB 205 (step S1504).

If the step counts for the two most recently measured time slots are respectively greater than or equal to a threshold defined in the threshold DB 205 (step S1504: YES), the estrus notifying apparatus 104 sets the estrus predictor flag to "1" and stores the flag ON date/time for the estrus predictor flag (step S1505). For example, the estrus notifying apparatus 104 stores as the flag ON date/time, the currently most recent date and time of measurement included in the received measurement result information. Further, for example, the estrus notifying apparatus 104 may obtain the current date and time and store the obtained date and time as the flag ON date/time.

The estrus notifying apparatus 104 determines whether thereafter, tracking start information is received within a given period (1 to 5 minutes) from the client apparatus 105 (step S1506). If tracking start information is received (step S1506: YES), the estrus notifying apparatus 104 transmits to the relay device 102, a change instruction for changing the transmission interval from a one-hour interval to a 10-minute interval (step S1507), and ends the series of operations according to the present flowchart.

On the other hand, if no tracking start information is received within the given period (step S1506: NO), the estrus notifying apparatus 104 ends the series of operations according to the present flowchart. Further, at step S1504, if the step counts are not greater than or equal to the threshold (step S1504: NO), the estrus notifying apparatus 104 ends the series of operations according to the present flowchart.

FIG. 16 is a flowchart (part 2) depicting an example of the process procedure of the estrus notifying apparatus 104. For example, the estrus notifying apparatus 104 performs the operation in the flowchart depicted in FIG. 16, if an estrus predictor flag is set to "1", i.e., ON, in the step count DB 202 and tracking start information has been received from the client apparatus 105.

In the flowchart depicted in FIG. 16, the estrus notifying apparatus 104 determines whether measurement result information, communications device identification information, and relay device identification information have been received from the relay device 102 (step S1601). The estrus notifying apparatus 104 stands by until measurement result information, communications device identification information, and relay device identification information are received (step S1601: NO).

Upon receiving measurement result information, communications device identification information, and relay device identification information (step S1601: YES), the estrus notifying apparatus 104, similar to step S1502, updates the stored contents of the transmission source DB 203, based on the received communications device identification information and relay device identification information (step S1602).

The estrus notifying apparatus 104 obtains based on the received relay device identification information and the relay device DB 204, the installation position information of the relay device 102 that transmitted the currently received measurement result information (step S1603). At step S1603, the estrus notifying apparatus 104 obtains from the relay device DB 204, the installation position information to which the relay device ID in the received relay device identification information is correlated.

The estrus notifying apparatus 104 transmits to the client apparatus 105, the relay device identification information received at step S1601 and the installation position information obtained at step S1603 (step S1604). The estrus notifying apparatus 104 calculates based on the received measurement result information, the step count of the livestock animal A having the communications device 101 that transmitted the measurement result information, and stores the calculated step count to the step count cumulation table 1100 (step S1605).

The estrus notifying apparatus 104 determines whether an hourly step count can be calculated based on measurement result information transmitted at 10-minute intervals (step S1606). For example, at step S1606, the estrus notifying apparatus 104 determines whether six step counts for cumulation are stored in step count cumulation table 1100. If six step counts for cumulation are stored, the estrus notifying apparatus 104 determines that an hourly step count can be calculated. If an hourly step count cannot be calculated (step S1606: NO), the estrus notifying apparatus 104 transitions to the operation at step S1601.

If an hourly step count can be calculated (step S1606: YES), the estrus notifying apparatus 104 sums six step counts for cumulation stored in the step count cumulation table 1100 to calculate an hourly step count, and stores the calculated step count into the step count DB 202 (step S1607). Thus, the estrus notifying apparatus 104 can calculate an hourly step count from measurement result information transmitted at 10-minute transmission intervals and can store the step count into the step count DB 202.

The estrus notifying apparatus 104 determines if the hourly step count calculated at step S1607 is greater than or equal to a threshold defined in the threshold DB 205 (step S1608). If the hourly step count is greater than or equal to the threshold (step S1608: YES), the estrus notifying apparatus 104 sets the estrus indication flag to "1", i.e., ON, and stores the flag ON date/time (step S1609). The estrus notifying apparatus 104 transmits to the communications device 101, via the relay device 102, a change instruction instructing the transmission interval to be changed from a 10-minute interval to a one-minute interval (step S1610), and ends the series of operations according to the present flowchart.

On the other hand, if the hourly step count calculated at step S1707 is not greater than or equal to the threshold (step S1608: NO), the estrus notifying apparatus 104 sets the estrus predictor flag to "0", i.e., OFF (step S1611). The estrus notifying apparatus 104 transmits to the communications device 101, via the relay device 102, a change instruction instructing the transmission interval to be changed from a 10-minute interval to a one-hour interval (step S1612), and ends the series of operations according to the present flowchart. For example, upon setting the estrus predictor flag to OFF at step S1711, the estrus notifying apparatus 104 clears the stored contents of the step count cumulation table 1100.

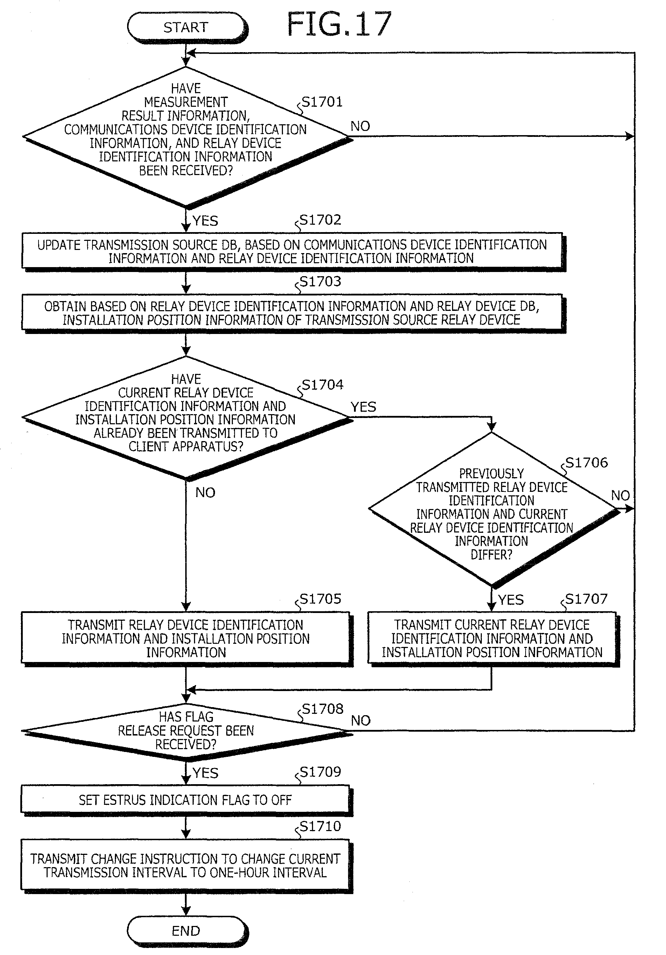

FIG. 17 is a flowchart (part 3) depicting an example of the process procedure of the estrus notifying apparatus 104. For example, the estrus notifying apparatus 104 performs the operations in the flowchart depicted in FIG. 17, if in the step count DB 202, an estrus indicator flag is set to "1", i.e., ON.

In the flowchart depicted in FIG. 17, the estrus notifying apparatus 104 determines whether measurement result information, communications device identification information, and relay device identification information has been received from the relay device 102 (step S1701). The estrus notifying apparatus 104 stands by until measurement result information, communications device identification information, and relay device identification information are received (step S1701: NO).

Upon receiving measurement result information, communications device identification information, and relay device identification information (step S1701: YES), the estrus notifying apparatus 104, similar to step S1602, etc., updates the stored contents of the transmission source DB 203, based on the received communications device identification information and relay device identification information (step S1702). The estrus notifying apparatus 104, similar to step S1603, obtains based on the received relay device identification information and the relay device DB 204, the installation position information of the relay device 102 that transmitted the currently received measurement result information (step S1703).

The estrus notifying apparatus 104 determines whether the same relay device identification information and installation position information of the relay device 102 have already been transmitted to the client apparatus 105 (step S1704). If so (step S1704: NO), i.e., this transmission is the first transmission after the last flag release, the estrus notifying apparatus 104 transmits to the client apparatus 105, via the network 220, the relay device identification information received at step S1701 and the installation position information received at step S1703 (step S1705).

If the relay device identification information and the installation position information of the relay device 102 have already been transmitted to the client apparatus (step S1704: YES), the estrus notifying apparatus 104 determines whether the previously transmitted relay device identification information and the current relay device identification information differ (step S1706).

If the relay device identification information differs (step S1706: YES), the estrus notifying apparatus 104 transmits to the client apparatus 105, via the network 220, the relay device identification information received at step S1701 and the installation position information received at step S1703 (step S1707). On the other hand, if the relay device identification information is identical (step S1706: NO), the estrus notifying apparatus 104 transitions to the operation at step S1701, and repeats the operations at the above steps.

The estrus notifying apparatus 104 determines whether a flag release request from the worker W and for setting the estrus indication flag to OFF has been received from the client apparatus 105 (step S1708). If no flag release request has been received (step S1708: NO), the estrus notifying apparatus 104 transitions to the operation at step S1701, and repeats the operations at the above steps.

On the other hand, if a flag release request has been received (step S1708: YES), the estrus notifying apparatus 104 sets the estrus indication flag, which is ON, to OFF (step S1709). The estrus notifying apparatus 104 transmits to the communications device 101, via the relay device 102, a change instruction instructing the current transmission interval to be changed to a one-hour interval (step S1710), and ends the series of operations according to the present flowchart. Thus, the estrus notifying apparatus 104 can return the transmission interval of the communications device 101 to a one-hour interval in response to an operation by the worker W, enabling battery consumption of the communications device 101 to be suppressed.

Similar to step S1707, the estrus notifying apparatus 104 may calculate an hourly step count based on measurement result information transmitted at one-minute intervals, and store the hourly step count to the step count DB 202. Thus, similar to a case where the communications device 101 transmits measurement result information at 10-minute intervals, in a case where the measurement result information is transmitted at one-minute intervals, the estrus notifying apparatus 104 can calculate the step count and store the step count to the step count DB 202.

As described, according to the estrus notifying apparatus 104 of the present embodiment, if an abnormal state of a livestock animal A is detected, the transmission interval for measurement result information of the communications device 101 can be shortened. Each time measurement result information is received at the shortened transmission interval, the worker W can be notified of the relay device ID of the relay device 102 that relayed the received measurement result information.

Thus, when no abnormal state of a livestock animal A is detected, the estrus notifying apparatus 104 can reduce the transmission frequency of the measurement result information of the communications device 101, enabling battery consumption of the communications device 101 to be suppressed and the work load of the worker W to be reduced. Further, if an abnormal state of a livestock animal A is detected, the estrus notifying apparatus 104 can shorten the interval at which the worker W is notified of the relay device identification information indicative of the location of the livestock animal A, can support the tracking of the livestock animal A in the abnormal state, by the worker W, and can reduce the work load of the worker W.

According to the estrus notifying apparatus 104, if estrus of a livestock animal A is detected and tracking start information is received from the worker W, the transmission interval for the measurement result information of the communications device 101 can be shortened. Further, according to the estrus notifying apparatus 104, when measurement result information is received at the shortened transmission interval and the relay device 102 has changed, the worker W can be notified of the relay device ID of the relay device 102 that relayed the received measurement result information. Thus, the estrus notifying apparatus 104 can suppress battery consumption of the communications device 101 and support the tracking of a livestock animal A in estrus by the worker W.

According to the estrus notifying apparatus 104, based on the step count for each differing time slot sequentially measured, whether a livestock animal A is in estrus can be determined. Thus, the estrus notifying apparatus 104 can increase the accuracy in detecting estrus of a livestock animal A and reduce the work load placed on the worker W consequent to errant judgment.

According to the estrus notifying apparatus 104, if a livestock animal A is detected to be in estrus and the worker W has started tracking, the transmission interval for the measurement result information of the communications device 101 can be further shortened. Thus, the estrus notifying apparatus 104 can reduce the distance that the livestock animal A can travel during the transmission interval for the measurement result information and support the tracking of the livestock animal A in estrus by the worker W.

According to the estrus notifying apparatus 104, if estrus of the livestock animal A has been determined to be errantly judged, the transmission interval of the communications device 101 can be returned to the usual transmission interval from the shortened transmission interval. Thus, the estrus notifying apparatus 104 can suppress battery consumption of the communications device 101 and reduce the work load of the worker W.

According to the estrus notifying apparatus 104, the worker W can be notified of the installation position information indicating the installation position of the relay device 102. Thus, the estrus notifying apparatus 104 can simplify the task of grasping the position of the livestock animal A by the worker W and facilitate support of the tracking of the livestock animal A.

In the description above, although an example has been given taking "estrus" as one example of a state change of livestock animals, the estrus notifying apparatus 104 may be used as an apparatus that gives notification of state changes including illness, giving birth, the appearance of a predator, etc.

The estrus notifying method described in the present embodiment may be implemented by executing a prepared program on a computer such as a personal computer and a workstation. The program is stored on a computer-readable recording medium such as a hard disk, a flexible disk, a CD-ROM, an MO, and a DVD, read out from the computer-readable medium, and executed by the computer. The program may be distributed through a network such as the Internet.

### EXPLANATIONS OF LETTERS OR NUMERALS

- 101: communications device
- 102: relay device
- 104: estrus notifying apparatus
- 105: client apparatus

## Claims

1. An estrus notifying method of giving notification of estrus, based on a step count measurement result that is reported through a relay device, at a first transmission interval, and by a step counting device attached to each livestock animal among a plurality of livestock animals, the estrus notifying method being executed by a computer and comprising:
receiving the step count measurement result of the livestock animal and information of the relay device used to report the step count measurement result;
determining with respect to each livestock animal and based on the step count measurement result, whether the livestock animal is in estrus;
notifying a worker of identification information of a specific livestock animal determined to be in estrus and position information correlated with a specific relay device used to report the step count measurement result of the specific livestock animal;
setting a specific step counting device that is attached to the specific livestock animal, to be a tracking target, when notification of attending to the specific livestock animal is received from the worker;
transmitting to the specific step counting device, a change instruction to change a reporting interval for the step count measurement result, to a second transmission interval that is shorter than the first transmission interval; and
notifying the worker of position information correlated with a new relay device, each time the specific relay device used by the specific step counting device to report the step count measurement result changes.

2. An estrus notifying apparatus that gives notification of estrus, based on a step count measurement result that is reported through a relay device, at a first transmission interval, and by a step counting device attached to each livestock animal among a plurality of livestock animals, the estrus notifying apparatus comprising:
a receiving unit that receives the step count measurement result of the livestock animal and information of the relay device used to report the step count measurement result;
a determining unit that determines with respect to each livestock animal and based on the step count measurement result, whether the livestock animal is in estrus;
a notifying unit that notifies a worker of identification information of a specific livestock animal determined to be in estrus and position information correlated with a specific relay device used to report the step count measurement result of the specific livestock animal;
a setting unit that sets a specific step counting device that is attached to the specific livestock animal, to be a tracking target, when notification of attending to the specific livestock animal is received from the worker; and
a transmitting unit that transmits to the specific step counting device, a change instruction to change a reporting interval for the step count measurement result, to a second transmission interval that is shorter than the first transmission interval, wherein
the notifying unit notifies the worker of position information correlated with a new relay device, each time the specific relay device used by the specific step counting device to report the step count measurement result changes.

3. An estrus notifying program that causes a computer to give notification of estrus, based on a step count measurement result that is reported through a relay device, at a first transmission interval, and by a step counting device attached to each livestock animal among a plurality of livestock animals, the estrus notifying program further causing the computer to execute:
receiving the step count measurement result of the livestock animal and information of the relay device used to report the step count measurement result;
determining with respect to each livestock animal and based on the step count measurement result, whether the livestock animal is in estrus;
notifying a worker of identification information of a specific livestock animal determined to be in estrus and position information correlated with a specific relay device used to report the step count measurement result of the specific livestock animal;
setting a specific step counting device that is attached to the specific livestock animal, to be a tracking target, when notification of attending to the specific livestock animal is received from the worker;
transmitting to the specific step counting device, a change instruction to change a reporting interval for the step count measurement result, to a second transmission interval that is shorter than the first transmission interval; and
notifying the worker of position information correlated with a new relay device, each time the specific relay device used by the specific step counting device to report the step count measurement result changes.
